# EUROPÄISCHE PATENTSCHRIFT

(11) **EP 2 204 441 B1**
(45) Veröffentlichungstag und Bekanntmachung des Hinweises auf die Patenterteilung: **13.07.2011**
(21) Anmeldenummer: 09178231.8
(22) Anmeldetag: 07.12.2009
(51) Int. Cl.: C12N 1/00, C12N 1/20

(54) **Verfahren zur fermentativen Herstellung von heterologen Proteinen mittels Escherichia coli**
Process for the fermentative production of heterologous proteins by means of Escherichia coli
Véhicule submersible commandé à distance avec terminal de montage d'amarre réglable

(30) Priorität: 19.12.2008 DE 102008063900
(43) Veröffentlichungstag der Anmeldung: 07.07.2010
(73) Patentinhaber: Wacker Chemie AG, 81737 München (DE)
(72) Erfinder: Daßler, Tobias, Dr., 81825 München (DE); Mitterweger, Simone, Dr., 82205 Gilching (DE); Wich, Günter, Dr., 81377 München (DE)
(74) Vertreter: Potten, Holger

(56) Entgegenhaltungen:
- WO-A1-98/59043
- DEDHIA N, RICHINS R, MESINA A, CHEN W.: "Improvement in recombinant protein production in ppGpp-deficient Escherichia coli." BIOTECHNILOGY AND BIOENGINEERING, Bd. 53, Nr. 4, 20. Februar 1997 (1997-02-20), Seiten 379-386, XP002574595
- FU Z B ET AL: "A two-stage refinement approach for the enhancement of excretory production of an exoglucanase from Escherichia coli", PROTEIN EXPRESSION AND PURIFICATION, ACADEMIC PRESS, SAN DIEGO, CA, vol. 48, no. 2, 1 August 2006 (2006-08-01) , pages 205-214, XP024908822, ISSN: 1046-5928 [retrieved on 2006-08-01]

## Beschreibung

Die Erfindung betrifft ein Verfahren zur fermentativen Herstellung von heterologen Proteinen mittels eines Escherichia coli-Stammes mit einer attenuierten (p)ppGpp-Synthetase II-Aktivität.

Der Markt für rekombinante Proteinpharmazeutika (Pharmaproteine/Biologics) ist in den letzten Jahren stark gewachsen. Besonders wichtige Proteinpharmazeutika sind eukaryontische Proteine, vor allem Säugetierproteine und menschliche Proteine. Beispiele wichtiger Pharmaproteine (pharmazeutisch aktive Proteine) sind Cytokine, Wachstumsfaktoren, Proteinkinase, Protein- und Peptidhormone sowie Antikörper und Antikörperfragmente. Aufgrund der immer noch sehr hohen Produktionskosten für Pharmaproteine wird laufend nach effizienteren und damit kostengünstigeren Verfahren und Systemen für deren Herstellung gesucht.

Generell werden rekombinante Proteine entweder in Säugerzellkulturen oder in mikrobiellen Systemen hergestellt. Mikrobielle Systeme weisen gegenüber Säugerzellkulturen den Vorteil auf, dass auf diese Weise rekombinante Proteine in kürzerer Zeit und zu geringeren Kosten hergestellt werden können. Für die Produktion von rekombinanten Proteinen eignen sich daher vor allem Bakterien. Aufgrund seiner sehr gut untersuchten Genetik und Physiologie, der kurzen Generationszeit und der einfachen Handhabung, ist das Gram-negative Enterobakterium Escherichia coli (E. coli) gegenwärtig der am häufigsten verwendete Organismus zur Produktion rekombinanter Proteine. Besonders bevorzugt sind Produktionsverfahren für rekombinante Proteine in E. coli, bei denen das Zielprotein in hoher Ausbeute und in der korrekten Faltung direkt ins Fermentationsmedium sekretiert wird.

In der Literatur sind eine Reihe von E. coli-Stämmen und Prozesse mit E. coli-Stämmen offenbart, durch die eine Sekretion von rekombinanten Proteinen ins Fermentationsmedium erreicht wird. So sind beispielsweise in US2008254511 verschiedene E. coli-Stämme beschrieben, die eine Mutation im Gen für das Braun'sche Lipoprotein (Lpp) aufweisen und die überproduzierte heterologe Proteine in das Medium ausschleusen.

Für die Regulation und Koordination des Stoffwechsels unter Bedingungen, wenn die Zelle in eine Mangelsituation an Aminosäuren oder der Energiequelle gerät, besitzt E. coli einen Mechanismus, der als "stringente Kontrolle" bezeichnet wird. Dabei spielen die Alarmone Guanosin-Tetraphosphat (ppGpp) und Guanosin-Pentaphosphat (pppGpp), im Folgenden allgemein als (p)ppGpp zusammengefasst, als regulatorische Signalmoleküle eine wichtige Rolle, u.a. dadurch, dass durch Erhöhung des (p)ppGpp-Spiegels in der Zelle die Synthese stabiler RNAs (rRNA, tRNA) gebremst und die Expression einiger Aminosäure-Biosynthese-Gene verstärkt wird (Cashel et al., 1996, Escherichia coli and Salmonella Cellular and Molecular Biology, ed. Neidhardt, F.C. ASM Press, Washington, D.C. pp. 1458-1496). E. coli besitzt zwei Enzyme für die Synthese von (p)ppGpp aus ATP und GDP (ppGpp) bzw. ATP und GTP (pppGpp): Die (p)ppGpp-Synthetase I (PSI), kodiert von dem relA-Gen, ist verantwortlich für die (p)ppGpp-Synthese im Rahmen der stringenten Kontrolle, wenn die Zellen einem Aminosäure-Mangel ausgesetzt sind. Wird das Wachstum der Zellen jedoch aufgrund der Erschöpfung der primären Kohlenstoff-Quelle verlangsamt, erfolgt die (p)ppGPP-Synthese vorwiegend durch die (p)ppGpp-Synthetase II (PSII), welche von dem spoT-Gen kodiert wird. Im Unterschied zur PSI hat das spoT-Genprodukt neben der (p)ppGpp-Synthetase-Aktivität auch noch eine (p)ppGpp-3'-Pyrophospho-hydrolase-Aktivität und kann demnach den (p)ppGpp-Spiegel der Zelle auch aktiv durch den Abbau dieser Verbindung steuern (Gentry und Cashel, 1996, Mol. Microbiol. 19, 1373-84). Es wurde gezeigt, dass die aktiven Zentren für die beiden unterschiedlichen katalytischen Aktivitäten des PSII-Enzyms zwar nicht identisch, aber in enger räumlicher Nachbarschaft im N-terminalen Bereich des Proteins lokalisiert sind.

Im Rahmen der vorliegenden Erfindung ist unter der Bezeichnung "PSII-Aktivität" die Aktivität eines Enzyms zu verstehen, welches einerseits die (p)ppGpp-Synthese und andererseits auch die (p)ppGpp-Hydrolyse katalysieren kann.

Sowohl relA-Mutanten als auch spoT-Mutanten von E. coli sind bekannt (Cashel et al., 1996, Escherichia coli and Salmonella Cellular and Molecular Biology, ed. Neidhardt, F.C. ASM Press, Washington, D.C. pp. 1458-1496). Stämme mit einer relA1-Mutation, bei der das Insertionselement IS2 in die relA-Gensequenz integriert ist (relA: :IS2), besitzen noch eine geringe PSI-Restaktivität, wohingegen relA-Deletionsmutanten (ΔrelA) keine PSI-Aktivität mehr aufweisen (Metzger et al., 1989, J. Biol. Chem. 264, 21146-52). Im Unterschied zu relA-Wildtyp-Stämmen, bei denen der Mechanismus der stringenten Kontrolle intakt ist ("stringenter" Phänotyp), zeigen relA-Mutanten mit einer verminderten oder fehlenden PSI-Aktivität bei Eintritt in eine Aminosäure-Mangelsituation einen sogenannten "relaxierten" Phänotyp. Dieser äußert sich u.a. darin, dass die Zelle über den Basalspiegel hinaus kein (p)ppGpp anhäufen kann und dass die Synthese stabiler RNA-Moleküle nicht gestoppt wird, sondern unvermindert weiterläuft. Unter dem Basalspiegel ist der aus der Literatur (Cashel et al., 1996, Escherichia coli and Salmonella Cellular and Molecular Biology, ed. Neidhardt, F.C. ASM Press, Washington, D.C. pp. 1458-1496) bekannnte Konzentrationsbereich für (p)ppGpp (10 - 30 pmol/A₄₅₀) für exponentiell wachsende Zellen unter nicht-limitierten Wachstumsbedingungen (d.h. vor Eintritt in eine Aminosäure-Mangelsituation) zu verstehen. Aus Gitter et al. (1995, Appl. Microbiol. Biotechnol. 43, 89-92) ist bekannt, dass die *relA*-Mutante E. coli CP79 im Vergleich zu dem *relA*-Wildtyp-Stamm CP78 unter künstlich induzierten Aminosäure-Mangelbedingungen in das Periplasma sekretierte Proteine wie die β-Lactamase oder das Interferonα1 vermehrt in das Kulturmedium ausscheidet.

Stämme mit einer Mutation im spoT-Gen weisen je nach Ausmaß der Beeinträchtigung der PSII-Aktivität charakteristische Defekte des (p)ppGpp-Metabolismus auf, u.a. i) einen erhöhten basalen ppGpp-Spiegel während des normalen (balancierten) Wachstums gepaart mit einer geringeren Wachstumsgeschwindigkeit, ii) einen höher induzierten ppGpp-Spiegel während der stringenten Antwort und iii) eine geringere ppGpp-Umsatzrate nach Ende der stringenten Antwort.

Die DNS-Sequenz des spoT-Gens von E. coli (SEQ ID No. 1) kodiert für das spoT-Protein mit der Sequenz SEQ ID No. 2. Das spoT-Gen wird als Teil eines Operons, das die fünf Gene gmkrpoZ-spoT-spoU-recG umfaßt, exprimiert. Die Expression des Operons und damit auch die spoT-Expression wird einerseits vom stromaufwärts des gmk-Gens gelegenen P1-Promotor und andererseits vom P2-Promotor, der in der für die C-terminal kodierenden Region des gmk-Gens lokalisiert ist, gesteuert (Cashel et al., 1996, Escherichia coli and Salmonella Cellular and Molecular Biology, ed. Neidhardt, F.C. ASM Press, Washington, D.C. pp. 1458-1496). Bei Stämmen mit einer spoT1-Mutation (SEQ ID No. 3) weist das spoT-Protein nach dem Asparaginsäure-Rest 84 eine Insertion der zwei Aminosäuren Glutamin und Asparaginsäure auf, sowie einen Histidin-Tyrosin-Austausch an Position 255 (Durfee et al., 2008, J. Bacteriol. 190, 2597-606). Darüber hinaus wurden auch eine Reihe von spoT-Insertions- bzw. - Deletionsmutanten beschrieben (Xiao et al., 1991, J. Biol. Chem. 266, 5980-90).

Aufgabe der Erfindung war die Bereitstellung eines fermentativen Verfahrens, zur Herstellung eines heterologen Proteins mittels eines E. coli-Stammes, bei dem das Protein in das Fermentationsmedium ausgeschieden wird.

Diese Aufgabe wird gelöst durch ein Verfahren, bei dem ein E. coli-Stamm in einem Fermentationsmedium kultiviert wird, wobei der E. coli-Stamm ein Gen kodierend für das heterologe Protein, funktionell verknüpft mit einer Signalsequenz kodierend für ein Signalpeptid, enthält und der E. coli-Stamm das heterologe Protein in das Fermentationsmedium ausscheidet, **dadurch gekennzeichent, dass** der E. coli-Stamm eine attenuierte (p)ppGpp-Synthetase II-Aktivität aufweist.

Unter einer attenuierten (p)ppGpp-Synthetase II-Aktivität eines E. coli-Stammes ist im Rahmen der vorliegenden Erfindung vorzugsweise zu verstehen, dass der E. coli-Stamm maximal 50%, bevorzugt maximal 20% der (p)ppGpp-Synthetase II-Aktivität (PSII-Aktivität) des E. coli-Stammes vor Modifikation seiner PSII-Aktivität, im folgenden Ausgangsstamm genannt, aufweist. Besonders bevorzugt ist unter einer attenuierten PSII-Aktivität eines Stammes das Fehlen der PSII-Aktivität in diesem Stamm zu verstehen.

Methoden zur Bestimmung der Synthetase- und Hydrolase-Aktivität des PSII-Enzyms eines Stammes sind dem Fachmann bekannt (Mechold et al., 1996, J. Bacteriol. 178, 1401-1411).

Verfahren zur Attenuierung der PSII-Aktivität in einem Mikroorganimenstamm sind im Stand der Technik bekannt. Die PSII-Aktivität kann beispielsweise dadurch attenuiert werden, dass eine Mutation (Substitution, Insertion oder Deletion einzelner oder mehrerer Nukleotide) in den Leserahmen des spoT-Gens eingebracht wird, welche dazu führt, dass die Aktivität des Enzyms verringert wird. Darüber hinaus kann die PSII-Aktivität einer Zelle auch dadurch attenuiert werden, dass die Expression des spoT-Gens verringert wird, indem mindestens ein für die Expressionsregulation notwendiges Element (z.B. Promotor, Enhancer, Ribosomen-Bindestelle) durch Substitution, Insertion oder Deletion einzelner oder mehrerer Nukleotide mutiert wird. Solche in ihrer Basensequenz von der Sequenz des spoT-Wildtyp-Gens auf Grund von Mutationen abweichenden DNS-Sequenzen werden im folgenden auch als spoT-Allele bezeichnet. Auch durch eine vollständige Deletion des spoT-Gens oder seiner regulatorischen Regionen aus dem Chromosom ist eine Attenuierung der PSII-Aktivität möglich.

Dem Fachmann sind Verfahren zur Erzeugung von spoT-Allelen bekannt. Allele des spoT-Gens kann man beispielsweise durch unspezifische oder gezielte Mutagenese mit der DNS des spoT-Wildtyp-Gens als Ausgangsmaterial herstellen. Unspezifische Mutationen innerhalb des spoT-Gens oder des Promotorbereichs des spoT-Gens können z.B. durch chemische Agentien wie Nitrosoguanidin, Ethylmethansulfonsäure u.ä. und/oder durch physikalische Methoden und/oder durch unter bestimmten Bedingungen durchgeführte PCR-Reaktionen erzeugt werden. Methoden zur Einführung von Mutationen an spezifischen Positionen innerhalb eines DNS-Fragmentes sind bekannt. So kann beispielsweise der Austausch einer oder mehrerer Basen in einem DNS-Fragment, das das spoT-Gen und dessen Promotorregion umfasst, mittels PCR unter Verwendung geeigneter Oligonukleotide als Primer erfolgen. Außerdem ist es möglich, das gesamte spoT-Gen bzw. ein neues spoT-Allel mittels Genynthese herzustellen.

spoT-Allele werden in der Regel zunächst in vitro erzeugt und anschließend in das Chromosom der Zelle eingebracht, wodurch das ursprünglich vorhandene spoT-Wildtyp-Gen ersetzt und somit eine spoT-Mutante des Stammes erzeugt wird. spoT-Allele können mittels bekannter Standardmethoden in das Chromosom einer Wirtszelle anstelle des spoT-Wildtyp-Gens/Promotors eingebracht werden. Dies kann beispielsweise mittels des in Link et al. (1997, J. Bacteriol. 179: 6228-37) beschriebenen Verfahrens zur Einführung chromosomaler Mutationen in ein Gen über den Mechanismus der homologen Rekombination erfolgen. Die Einführung einer chromosomalen Deletion des gesamten spoT-Gens oder eines Teils davon ist beispielsweise mit Hilfe des λ-Red Rekombinase-Systems nach der von Datsenko und Wanner (2000, Proc. Natl. Acad. Sci. U S A. 97: 6640-5) beschriebenen Methode möglich. spoT-Allele können auch über eine Transduktion mittels P1-Phagen oder Konjugation aus einem Stamm mit spoT-Mutation auf einen spoT-Wildtyp-Stamm übertragen werden, wobei das spoT-Wildtyp-Gen im Chromosom gegen das entsprechende spoT-Allel ersetzt wird. Neben den beschriebenen E. coli-Stämmen mit einer spoT-Mutation sind dem Fachmann auch Methoden zur Erzeugung von spoT-Mutanten beliebiger E. coli-Stämme bekannt.

Eine bevorzugte spoT-Mutation, ist die spoT1-Mutation oder eine spoT-Mutation, die zu einer als bevorzugt definierten attenuierten PSII-Aktivität führt. Besonders bevorzugt ist eine Mutation, die den vollständigen Verlust der PSII-Aktivität bewirkt.

Bevorzugt weist der E. coli-Stamm, der im erfindungsgemäßen Verfahren Verwendung findet, neben der attenuierten PSII-Aktivität eine Mutation im relA-Gen auf, welche zu einem relaxierten Phänotyp führt.

Eine bevorzugte relA-Mutation, ist beispielsweise die relA1-Mutation oder eine relA-Mutation, die einen vollständigen Verlust der PSI-Aktivität bewirkt.

Ebenfalls bevorzugt weist der E. coli-Stamm, der im erfindungsgemäßen Verfahren Verwendung findet, neben der attenuierten PSII-Aktivität eine "leaky"-Mutation auf. Unter einer "Leaky-Mutation" ist eine Mutation in Genen für Stukturelemente der äußeren Zellmembran oder der Zellwand, ausgewählt aus der Gruppe der omp-Gene, tol-Gene, excD-Gen, excC-Gen, lpp-Gen, pal-Gen, env-Gene und lky-Gene zu verstehen, die dazu führt, dass die Zellen vermehrt periplasmatische Proteine ins Medium abgeben (Shokri et al., Appl. Microbiol. Biotechnol. 60 (2003), 654-664). Vorzugsweise handelt es sich um eine "leaky"-Mutation im lpp-Gen, besonders bevorzugt um eine Mutation ausgewählt aus der Gruppe lpp1-Mutation, lpp3-Mutation und lpp-Deletionsmutation. Eine lpp1-Mutation ist eine Mutationen im lpp-Gen, die zu einem Austausch des Arginin-Rests an Position 77 gegen einen Cystein-Rest führt, eine lpp3-Mutation ist eine Mutationen im lpp-Gen, die zu einem Austausch des Glycin-Rests an Position 14 gegen einen Asparaginsäure-Rest führt. Diese Mutationen sind in US2008254511 detailiert beschrieben. Bei der lpp-Deletionsmutation handelt es sich vorzugsweise um eine Deletion von mindestens einem Nukleotid im lpp-Gen selbst oder in der Promotorregion des lpp-Gens, welche dazu führt, dass die Zellen eine erhöhte Leakiness für periplasmatische Proteine aufweisen.

Unter erhöhter Leakiness ist im Sinne der vorliegenden Erfindung zu verstehen, dass nach einer Fermentation der Zellen eine höhere Konzentration von periplasmatischen Proteinen, z. B. der Alkalischen Phosphatase, im Nährmedium vorliegt, als bei einer Fermentation des E. coli-Stammes W3110 (ATCC 27325) unter den gleichen Bedingungen.

Insbesondere bevorzugt sind E. coli-Stämme die neben der attenuierten PSII-Aktivität sowohl einen relaxierten Phänotyp als auch eine "leaky"-Mutation aufweisen, wobei auch hier die jeweils genannten bevorzugten Ausführungsformen besonders bevorzugt sind.

Unter einem heterologen Protein ist im Sinne der vorliegenden Erfindung ein Protein zu verstehen, das nicht zum Proteom, d.h. der gesamten natürlichen Protein-Ausstattung, eines E. coli K12-Stammes gehört. Alle natürlicherweise in E. coli K12-Stämmen vorkommenden Proteine lassen sich aus der bekannten Genomsequenz von E. coli K12 ableiten (Genbank Accession No. NC_000913). Ein in E. coli produziertes heterologes Protein weist mehr als 50%, bevorzugt mehr als 70%, besonders bevorzugt mehr als 90% der spezifischen Aktivität bzw. der Wirkung (Funktion) auf, die charakteristisch für das jeweilige heterologe Protein unter natürlichen Bedingungen in der homologen Wirtszelle, oder aus homologen Wirtszellen gewonnen, ist.

Bevorzugt handelt es sich bei dem heterologen Protein um ein eukaryontisches Protein, besonders bevorzugt um ein Protein, welches eine oder mehrere Disulfid-Brücken enthält oder welches in seiner funktionellen Form als Di- oder Multimer vorliegt, d.h. dass das Protein eine Quartärstruktur besitzt und aus mehreren identischen (homologen) oder nicht-identischen (heterologen) Untereinheiten aufgebaut ist.

Zu den wichtigsten heterologen Proteinklassen zählen Antikörper und deren Fragmente, Cytokine, Wachstumsfaktoren, Proteinkinasen, Proteinhormone, Lipokaline, Antikaline, Enzyme, Bindeproteine und molekulare Scaffolds und davon abgeleitete Proteine. Beispiele für diese Proteinklassen sind u.a. heavychain Antikörper und deren Fragmente (z.B. Nanobodies), einzelkettige Antikörper, Interferone, Interleukine, Interleukinrezeptoren, Interleukinrezeptor Antagonisten, G-CSF, GM-CSF, M-CSF, Leukämieinhibitoren, Stammzellenwachstumsfaktoren, Tumornekrosefaktoren, Wachstumshormone, insulinartige Wachstumsfaktoren, Fibroblastenwachstumsfaktoren, von Blutplättchen abstammende Wachstumsfaktoren, transformierende Wachstumsfaktoren, Hepatocyten-Wachstumsfaktoren, knochenmorphogenetische Faktoren, Nervenwachstumsfaktoren, vom Gehirn abstammende neurotrophe Faktoren (BDNF), von Gliazelllinien abstammende neurotrophe Faktoren, Angiogenese-Inhibitoren, Gewebeplasminogen-Aktivatoren, Blutgerinnungsfaktoren, Trypsin-Inhibitoren, Elastase-Inhibitoren, Komplementbestandteile, hypoxie-induzierte Stressproteine, Proto-Oncogen-Produkte, Transkriptionsfaktoren, viruskonstitutive Proteine, Proinsulin, Prourokinase, Erythropoietin, Thrombopoietin, Neurotrophin, Protein C, Glucocerebrosidase, Superoxid-Dismutase, Renin, Lysozym, P450, Prochymosin, Lipocortin, Reptin, Serumalbumin, Streptokinase, Tenecteplase, CNTF und Cyclodextrin-Glycosyl-Transferasen. Beispiele für von molekularen Scaffolds abgeleiteten Proteinen sind u.a. Evibodies (abgeleitet von CTLA-4), Affibodies (von Protein A von *S. aureus*), Avimere (von humaner A-Domäne Familie), Transbodies (von Transferrin), DARPins (vom Ankyrin Repeat Protein), Adnectin (von Fibronectin III), Peptid Aptamere (von Thioredoxin), Microbodies (von Microprotein), Affiline (von Ubiquitin), α-Crystallin, Charybdotoxin, Tetranectin, PDZ Domäne des RAS-bindenden Proteins AF-6, Kunitz-Typ Domäne von Proteininhibitoren.

Eine besonders wichtige Klasse von Proteinen, die aus mehreren Proteinuntereinheiten besteht, sind Antikörper. Antikörper werden in der Forschung, in der Diagnostik und als Therapeutikum in großem Umfang eingesetzt, so dass besonders effiziente und in technischem Ausmaß mögliche Produktionsverfahren notwendig sind.

Auch funktionelle Fab-Antikörperfragmente und Volllängen-Antikörper können mittels des erfindungsgemäßen Verfahrens extrazellulär hergestellt werden. Bevorzugte Volllängen-Antikörper sind dabei Antikörper der IgG- und IgM-Klasse, inbesondere der IgG-Klasse.

Bei der Herstellung funktioneller Fab-Antikörperfragmente muss die Zelle das entsprechende Fragment der leichten Kette (LC), welche die Domänen V_{L} und C_{L} umfasst, und der schweren Kette (HC), welche die Domänen V_{H} und CH1 umfasst, gleichzeitig synthetisieren und dann in das Periplasma und schließlich in das Fermentationsmedium sekretieren. Außerhalb des Cytoplasmas erfolgt dann die Assemblierung der beiden Ketten zum funktionellen Fab-Fragment.

Analog zur Herstellung der Fab-Fragmente muss die Zelle die leichte und die schwere Kette eines Volllängen-Antikörpers gleichzeitig synthetisieren und dann in das Periplasma und schließlich in das Fermentationsmedium sekretieren. Außerhalb des Cytoplasmas erfolgt dann die Assemblierung der beiden Ketten zum funktionellen Volllängen-Antikörper.

Für die Sekretion von Proteinen aus dem Cytoplasma in das Periplasma ist es notwendig, das 5'-Ende des Gens des zu produzierenden Proteins *in frame* mit dem 3'-Ende einer Signalsequenz für den Protein-Export zu verknüpfen. Dazu sind prinzipiell die Gene aller Signalsequenzen geeignet, die in E.coli eine Translokation des Zielproteins mit Hilfe des Sec-Apparats ermöglichen. Verschiedene Signalsequenzen sind im Stand der Technik beschrieben, so z. B. die Signalsequenzen der folgenden Gene: phoA, ompA, pelB, ompF, ompT, lamB, malE, Staphylococcal protein A, StII und andere (Choi and Lee, Appl. Microbiol. Biotechnol. 64 (2004), 625-635).

Erfindungsgemäß bevorzugt ist die Signalsequenz des phoA- oder des ompA-Gens von E. coli oder die Signalsequenz für eine Cyclodextrin-Glycosyltransferase (CGTase) aus Klebsiella pneumoniae M5a1, oder die von dieser Signalsequenz abgeleitete Sequenz, die in US2008076157 offenbart ist. Besonders bevorzugt ist die in EP0448093 offenbarte Signalsequenz für eine Cyclodextrin-Glycosyltransferase (CGTase) aus Klebsiella pneumoniae M5a1 mit der Sequenz SEQ ID No. 4 sowie die davon abgeleitete Sequenz mit der SEQ ID No. 5, die in US2008076157 offenbart ist.

Die Herstellung des DNS-Moleküls, das eine *in frame*-Fusion aus einer Signalsequenz und dem Gen des rekombinanten Zielproteins umfasst, erfolgt nach dem Fachmann bekannten Methoden. So kann das Gen des Zielproteins zunächst mittels PCR unter Verwendung von Oligonukleotiden als Primer amplifiziert und anschließend mit gängigen molekularbiologischen Techniken mit dem DNS-Molekül, das die Sequenz eines Signalpeptids umfasst und das auf analoge Weise wie das Gen des Zielproteins erzeugt wurde, derart verknüpft werden, dass eine *in frame*-Fusion, d.h. ein durchgehender Leserahmen umfassend die Signalsequenz und das Gen des Zielproteins, entsteht. Alternativ kann auch das gesamte DNS-Molekül, das beide oben genannten funktionellen Abschnitte umfasst, mittels Gensynthese hergestellt werden. Diese Signalsequenz-rekombinantes Gen-Fusion kann dann entweder in einen Vektor, z.B. ein Plasmid, eingebracht werden, welches dann in die Wirtszelle mittels Transformation eingebracht wird, oder direkt nach bekannten Methoden in das Chromosom der Wirtszelle integriert werden. Bevorzugt wird die Signalsequenz-rekombinantes Gen-Fusion in ein Plasmid eingebracht und die Wirtszelle wird mit diesem Plasmid transformiert.

Für die Sekretion eines aus mehreren unterschiedlichen Untereinheiten bestehenden Proteins aus dem Cytoplasma in das Periplasma ist es notwendig, die Gene aller zu produzierenden Untereinheiten (Zielgene) jeweils funktionell mit einer Signalsequenz für den Protein-Export zu verknüpfen. Dabei können die Gene der verschiedenen Untereinheiten mit unterschiedlichen Signalsequenzen verknüpft werden oder der gleichen. Bevorzugt ist die Verknüpfung mit unterschiedlichen Signalsequenzen, besonders bevorzugt ist die Verknüpfung einer Untereinheit mit der Signalsequenz des phoA- oder ompA-Gens von E. coli und die Verknüpfung einer weiteren Untereinheit mit der Signalsequenz für eine Cyclodextrin-Glycosyltransferase (CGTase) aus Klebsiella pneumoniae M5a1 mit der Sequenz SEQ ID No. 4 (EP0448093) oder davon abgeleitete Sequenzen wie zum Beispiel die Sequenz SEQ ID No. 5 (US2008076157).

Die Signalsequenz-Zielgen-Fusionen der einzelnen Untereinheiten können dann entweder in einen Vektor, z.B. ein Plasmid, eingebracht werden, oder direkt nach bekannten Methoden in das Chromosom der Wirtszelle integriert werden. Dabei können die Signalsequenz-Zielgen-Fusionen der einzelnen Untereinheiten auf getrennten, aber miteinander kompatiblen Plasmiden kloniert werden oder sie können auf einem Plasmid kloniert werden. Die Genfusionen können dabei in einem Operon zusammengefasst werden oder sie können in jeweils separaten Cistronen exprimiert werden. Bevorzugt ist hier ein Zusammenfassen in einem Operon. Genauso können die beiden Genkonstrukte in einem Operon zusammengefasst oder in jeweils separaten Cistronen in das Chromosom der Wirtszelle integriert werden. Bevorzugt ist auch hier ein Zusammenfassen in einem Operon.

Vorzugsweise wird das DNS-Expressionskonstrukt (Signalsequenz-Zielgen-Fusion) bestehend aus einer Signalsequenz und einem rekombinanten Gen kodierend das zu sekretierende Protein, mit in E. coli funktionellen Expressionssignalen versehen (Promotor, Transkriptions-, Translationsstart, Ribosomenbindestelle, Terminator).

Als Promotoren eignen sich alle dem Fachmann bekannten Promotoren, wie einerseits beispielsweise induzierbare Promotoren wie der lac-, tac-, trc-, lambda PL, ara- oder tet-Promotor oder davon abgeleitete Sequenzen. Andererseits kann auch eine dauerhafte Expression erfolgen durch Verwendung eines konstitutiven Promotors, wie z. B. des GAPDH-Promotors. Es kann aber auch der normalerweise mit dem Gen des zu produzierenden rekombinanten Proteins verknüpfte Promotor Verwendung finden. Dieses Expressionskonstrukt (Promoter-Signalsequenz-rekombinantes Gen) für das zu produzierende Protein wird danach unter Anwendung von dem Fachmann bekannten Methoden (z.B. Transformation) in die Zellen mit einer attenuierten PSII-Aktivität eingebracht.

Dies erfolgt z.B. auf einem Vektor, z.B. einem Plasmid wie etwa einem Abkömmling von bekannten Expressionsvektoren wie pJF118EH, pKK223-3, pUC18, pBR322, pACYC184, pASK-IBA3 oder pET. Als Selektionsmarker für Plasmide geeignet sind Gene, die für eine Resistenz gegenüber z. B. Ampicillin, Tetracyclin, Chloramphenicol, Kanamycin oder andere Antibiotika codieren.

Erfindungsgemäß wird somit vorzugsweise ein E. coli-Stamm eingesetzt, in dem das rekombinante Gen funktionell verknüpft mit einer Signalsequenz kodierend für ein in E. coli aktives Signalpeptid, vorzugsweise ferner mit in E. coli funktionellen Expressionssignalen, vorzugsweise einem Promotor, einem Transkriptions-, Translationsstart, einer Ribosomenbindestelle, und einem Terminator versehen ist. Es handelt sich dabei vorzugsweise um die vorstehend genannten Expressionssignale.

Die Kultivierung (Fermentation) der Zellen mit einer attenuierten PSII-Aktivität, die ein DNS-Expressionskonstrukt bestehend aus einer Signalsequenz und einem rekombinanten Gen kodierend das zu sekretierende Protein, verknüpft mit funktionellen Expressionssignalen enthalten, erfolgt nach üblichen, dem Fachmann bekannten Fermentationsverfahren in einem Bioreaktor (Fermenter).

Die Fermentation findet vorzugsweise in einem üblichen Bioreaktor, beispielsweise einem Rührkessel, einem Blasensäulen-Fermenter oder einem Airlift-Fermenter statt. Besonders bevorzugt ist ein Rührkessel-Fermenter.

Bei der Fermentation werden die Zellen des Protein-Produktionsstammes in einem Flüssigmedium über einen Zeitraum von 16-150 h kultiviert, wobei verschiedene Parameter wie z.B. die Nährstoff-Zufuhr, der Sauerstoff-Partialdruck, der pH-Wert und die Temperatur der Kultur laufend kontrolliert und genau gesteuert werden. Der Zeitraum der Kultivierung beträgt vorzugsweise 24-72 h.

Als Fermentationsmedien kommen in Prinzip alle gängigen, dem Fachmann bekannten Medien für die Kultivierung von Mikroorganismen in Frage.

Dabei können Komplexmedien oder Minimalsalzmedien, denen ein definierter Anteil von Komplex-Komponenten wie z.B. Pepton, Trypton, Hefeextrakt, Melasse oder Corn Steep Liquor zugesetzt wird, benützt werden. Bevorzugt für die Herstellung von Pharmaproteinen sind chemisch definierte Salzmedien, also Medien, die im Gegensatz zum Vollmedium eine genau definierte Substrat-Zusammensetzung besitzen.

Im erfindungsgemäßen Verfahren wächst der E. coli Stamm mit einer attenuierten PSII-Aktivität sowie einem Gen kodierend ein heterologes Protein, welches *in frame* mit einer Signalsequenz kodierend für ein in E. coli funktionelles Signalpeptid verbunden ist, in einer zu einem Stamm mit einer PSII-Wildtyp-Aktivität vergleichbar kurzen Fermentationszeit zu vergleichbar hohen Zelldichten heran und sekretiert dabei das heterologe Protein in das Salzmedium.

Als primäre Kohlenstoffquelle für die Fermentation können prinzipiell alle von den Zellen verwertbaren Zucker, Zuckeralkohole oder organische Säuren bzw. deren Salze verwendet werden. Dabei werden bevorzugt Glucose, Laktose oder Glycerin eingesetzt. Besonders bevorzugt sind Glucose und Laktose. Auch ist eine kombinierte Fütterung mehrerer verschiedener Kohlenstoffquellen möglich. Die Kohlenstoffquelle kann dabei zu Beginn der Fermentation vollständig im Fermentationsmedium vorgelegt werden oder es wird nichts oder nur ein Teil der Kohlenstoffquelle zu Beginn vorgelegt und die Kohlenstoffquelle wird über den Verlauf der Fermentation zugefüttert. Besonders bevorzugt ist dabei eine Ausführungsform, bei der ein Teil der Kohlenstoffquelle vorgelegt wird und ein Teil gefüttert wird. Besonders bevorzugt wird die Kohlenstoffquelle in einer Konzentration von 10-30 g/l vorgelegt, die Fütterung gestartet, wenn die Konzentration auf kleiner 5 g/l abgefallen ist und so gestaltet, dass die Konzentration unter 5 g/l gehalten wird.

Der Sauerstoff-Partialdruck (pO₂) in der Kultur beträgt vorzugsweise zwischen 10 und 70% Sättigung betragen. Bevorzugt wird ein pO₂ zwischen 30 und 60 %, besonders bevorzugt liegt der pO₂ zwischen 45 und 55 % Sättigung.

Der pH-Wert der Kultur liegt vorzugsweise zwischen pH 6 und pH 8. Bevorzugt wird ein pH-Wert zwischen 6,5 und 7,5 eingestellt, besonders bevorzugt wird der pH-Wert der Kultur zwischen 6,8 und 7,2 gehalten.

Die Temperatur der Kultur beträgt vorzugsweise zwischen 15 und 45 °C. Bevorzugt ist ein Temperaturbereich zwischen 20 und 40 °C, besonders bevorzugt ist ein Temperaturbereich zwischen 25 und 35 °C, ganz besonders bevorzugt sind 30°C.

Die Aufreinigung des sekretierten Proteins aus dem Rohprodukt kann durch übliche und dem Fachmann bekannte Aufreinigungsmethoden geschehen wie sie im Stand der Technik bekannt sind. Üblicherweise werden in einem ersten Schritt durch Separationsmethoden wie Zentrifugation oder Filtration die Zellen von dem sekretierten Zielprotein abgetrennt. Das Zielprotein kann dann z. B. durch Ultrafiltration aufkonzentriert werden und wird dann über Standardmethoden, wie Fällung, Chromatographie oder Ultrafiltration weiter gereinigt. Besonders bevorzugt sind hierbei Methoden, wie Affinitätschromatographie, bei der die bereits korrekt gefaltete native Konformation des Proteins ausgenützt wird.

Die folgenden Beispiele dienen der weiteren Erläuterung der Erfindung.

Sämtliche eingesetzten molekularbiologischen und mikrobiologischen Verfahren wie Polymerase-Ketten-Reaktion (PCR), Gensynthese, Isolierung und Reinigung von DNS, Modifikation von DNS durch Restriktionsenzyme, Klenow-Fragment und Ligase, Transformation, P1-Transduktion etc., wurden in der dem Fachmann bekannten, in der Literatur beschriebenen oder von den jeweiligen Herstellern empfohlenen Art und Weise durchgeführt.

### Beispiel 1: Erzeugung einer chromosomalen relA-Deletionsmutante aus einem E. coli Wildtyp-Stamm (Vergleichsbeispiel)

Nächster Stand der Technik für die vorliegende Erfindung ist ein E. coli-Stamm, der aufgrund einer relA-Mutation eine verminderte PSI-Aktivität aufweist und der im Vergleich zu einem relA-Wildtyp-Stamm in das Periplasma sekretierte Proteine vermehrt in das Kulturmedium ausscheidet, wie er von Gitter et al. (1995, Appl. Microbiol. Biotechnol. 43, 89-92) beschrieben wurde. Um den Vorteil von Stämmen mit einer attenuierten PSII-Aktivität gegenüber diesem Stand der Technik deutlich zu machen, wurde eine relA-Mutante generiert.

Dazu wurde der *E. coli* Wildtyp-Stamm W3110 (American Type Culture Collection (ATCC) No. 27325) mit Hilfe der λ-Red-Rekombinase nach der Methode von Datsenko und Wanner (2000, Proc. Natl. Acad. Sci. U S A. 97: 6640-5) mutiert. Zunächst wurde mit Hilfe der Polymerase-Kettenreaktion (PCR) unter Verwendung der Oligonukleotide relAmut-fw (SEQ ID No. 6) und relAmut-rev (SEQ ID No. 7) als Primern und dem Plasmid pKD3 (Coli Genetic Stock Center (CGSC) No. 7631) als Matrize ein lineares DNS-Fragment erzeugt, welches ein Chloramphenicol-Resistenzgen enhält, und das von jeweils 50 Basenpaaren der 5'-Region- bzw. der 3'-Region des relA-Gens flankiert ist. Der Stamm W3110 wurde zuerst mit dem Plasmid pKD46 (CGSC No. 7739) transformiert. Kompetente Zellen des so erhaltenen Stammes W3110 pKD46, welche nach den Angaben von Datsenko und Wanner hergestellt worden waren, wurden mit dem o.g. per PCR erzeugten linearen DNS-Fragment transformiert. Die Selektion auf Integration der Chloramphenicol-Resistenzkassette in das Chromosom von W3110 an der Position des relA-Gens erfolgte auf LB-Agar-Platten, die 20 mg/l Chloramphenicol enthielten. Auf diese Weise wurden Zellen erhalten, bei denen 95 % der kodierenden Region des relA-Gens entfernt und durch die Chloramphenicol-Resistenzkassette ersetzt worden war. Dass die Integration an der korrekten Position im Chromosom erfolgte, wurde mittels PCR unter Verwendung der Oligonukleotide relA-fw (SEQ ID No. 8) und relA-rev (SEQ ID No. 9) und chromosomaler DNS der Chloramphenicol-resistenten Zellen als Matrize bestätigt. Die Zellen wurden von dem Plasmid pKD46 nach der beschriebenen Prozedur (Datsenko und Wanner) kuriert und der auf diese Weise erzeugte Stamm wurde mit W3110relA::cat bezeichnet.

Die Entfernung der Chloramphenicol-Resistenzkassette aus dem Chromosom des Stammes W3110relA::cat erfolgte nach der Vorschrift von Datsenko und Wanner mit Hilfe des Plasmids pCP20 (CGSC No. 7629), welches das FLP-Rekombinase-Gen trägt. Die schließlich mit dieser Prozedur erhaltene Chloramphenicolsensitive relA-Deletionsmutante von W3110 wurde mit W3110ΔrelA bezeichnet.

### Beispiel 2: Erzeugung einer chromosomalen spoT-Deletionsmutante aus einem E. coli ΔrelA-Stamm

Die Generierung einer spoT-Deletionsmutante aus dem *E*. *coli-*Stamm W3110ΔrelA erfolgte prinzipiell so wie für die Erzeugung des Stammes W3110ΔrelA in Beispiel 1 beschrieben.

Für die Erzeugung des linearen DNS-Fragments, das ein Chloramphenicol-Resistenzgen enhält, welches von jeweils 50 Basenpaaren der 5'-Region- bzw. der 3'-Region des spoT-Gens flankiert ist, wurden die Oligonukleotide spoTmut-fw (SEQ ID No. 10) und spoTmut-rev (SEQ ID No. 11) als Primer und das Plasmid pKD3 als Matrize eingesetzt. Der Stamm W3110ΔrelA/pKD46 wurde mit dem linearen DNS-Fragment transformiert. Der erfolgreiche Austausch des spoT-Gens im Chromosom von W3110ΔrelA gegen die Chloramphenicol-Resistenzkassette wurde mittels PCR unter Verwendung der Oligonukleotide spoT-fw (SEQ ID No. 12) und spoT-rev (SEQ ID No. 13) und chromosomaler DNS der Chloramphenicol-resistenten Zellen als Matrize bestätigt. Durch Entfernung der Chloramphenicol-Resistenzkassette aus dem Chromosom mittels der in Beispiel 1 beschrieben Prozedur wurde der Stamm W3110ΔrelAΔspoT erhalten.

### Beispiel 3: Einbringen einer chromosomalen spoT1-Mutation in verschiedene E. coli-Stämme

Alternativ zur ΔspoT-Mutante von W3110ΔrelA wurde eine Mutante von W3110ΔrelA mit dem spoT1-Allel (SEQ ID No 3; Durfee et al., 2008, J. Bacteriol. 190, 2597-606) erzeugt. Dazu wurde das spoT1-Allel aus dem Stamm K10 (Hfr PO2A tonA22 ompF626 re-1A1 pit-10 spoT1 T2(R); CGSC No. 4234) mittels Transduktion unter Verwendung des Phagen P1 auf den Stamm W3110ΔrelA übertragen. Zu diesem Zweck wurde zunächst eine Chloramphenicol-Resistenzkassette in das Chromosom von K10 ca. 2900 Basenpaare jenseits des spoT-Gens zwischen die Gene recG und gltS integriert (recG-gltS::cat). Dabei wurde prinzipiell so vorgegangen wie in Beispiel 1 beschrieben. Ein lineares DNS-Fragment, welches eine Chloramphenicol-Resistenzkassette enthält, die jeweils von 50 Basenpaaren der recG-gltS intergenen Region flankiert ist, wurde mittels PCR unter Verwendung der Oligonukleotide recG-catfw (SEQ ID No. 14) und gltS-catrev (SEQ ID No. 15) als Primern und dem Plasmid pKD3 als Matrize erzeugt und anschließend in den Stamm K10/pKD46 transformiert. Auf diese Weise wurden Chloramphenicol-resistente Klone erhalten, bei denen die Resistenzkassette in das Chromosom zwischen die Gene recG und gltS integriert ist (K10recG-gltS::cat). Dass die Integration an der korrekten Position erfolgte, wurde mittels PCR unter Verwendung der Oligonukleotide recG-fw (SEQ ID No. 16) und gltS-rev (SEQ ID No. 17) und chromosomaler DNS der Chloramphenicol-resistenten Zellen als Matrize bestätigt. Von dem Stamm K10recG-gltS::cat wurde gemäß Stand der Technik ein P1-Lysat erzeugt, mit dem dann der Stamm W3110ΔrelA infiziert wurde. Auf diese Weise wurden Chloramphenicol-resistente Transduktanden von W3110ΔrelA erhalten. Bei dem Transduktionsvorgang sollte mit einer theoretischen Co-Transduktionsrate von nahezu 100% neben der Resistenzkassette auch das auf dem Chromosom in enger räumlicher Nachbarschaft lokalisierte spoT1-Allel von K10recG-gltS::cat auf W3110ΔrelA übertragen werden. Dass dies tatsächlich der Fall war, wurde nach Amplifizierung des spoT-Gens mittels PCR unter Verwendung der Oligonukleotide spoT-fw (SEQ ID No. 12) und spoT-rev (SEQ ID No. 13) und chromosomaler DNS des Stammes als Matrize und anschließender Sequenzierung des PCR-Produkts bestätigt. Durch Entfernung der Resistenzkassette aus dem Chromosom des Stammes W3110ΔrelAspoT1recG-gltS::cat mittels der in Beispiel 1 beschrieben Prozedur wurde schließlich der Stamm W3110ΔrelAspoT1 erhalten.

Durch analoges Vorgehen wurde das spoT1-Allel außerdem auch in den *E. coli* Wildtyp-Stamm W3110 eingebracht. Der resultierende Stamm wurde mit W3110spoT1 bezeichnet.

### Beispiel 4: Einbringen von lpp-"leaky"-Mutationen in E. coli-Stämme mit einer attenuierten PSII-Aktivität

Einbringen einer lpp3-Mutation: Der Austausch des lpp-Wildtyp-Gens im Chromosom der Stämme W3110ΔrelAΔspoT bzw. W3110ΔrelAspoT1 gegen das lpp3-Allel erfolgte über homologe Rekombination. Zur Erzeugung der Stämme W3110ΔrelAΔspoTlpp3 bzw. W3110ΔrelAspoT1lpp3 wurde analog vorgegangen wie in Beispiel 3 von US2008254511 beschrieben.

Die auf diese Weise erhaltenen Stämme wurden mit W3110ΔrelAΔspoTlpp3 bzw. W3110ΔrelAspoT1lpp3 bezeichnet.

Einbringen einer lpp-Deletionsmutation: Das Einbringen einer lpp-Deletion in das Chromosom der Stämme

W3110ΔrelAΔspoT bzw. W3110ΔrelAspoT1 erfolgte mit Hilfe des λ-Red-Rekombinase-Systems nach der Methode von Datsenko und Wanner (2000, Proc. Natl. Acad. Sci. U S A. 97: 6640-5) und wurde analog zu Beispiel 1 von US2008254511 durchgeführt.

Die auf diese Weise erhaltenen Stämme wurden mit W3110ΔrelAΔspoTΔlpp bzw. W3110ΔrelAspoT1Δlpp bezeichnet.

### Beispiel 5: Fermentative Produktion einer Cyclodextrin-Glycosyl-Transferase im 10 1-Maßstab mit Stämmen, die eine attenuierte PSII-Aktivität aufweisen

Für die Produktion der Cyclodextrin-Glycosyl-Transferase (CGTase) aus Klebsiella pneumoniae M5a1 im 10 l-Maßstab wurden die Stämme W3110ΔrelA, W3110spoT1, W3110ΔrelAΔspoT, W3110ΔrelAspoT1, W3110ΔrelAΔspoTΔlpp, W3110ΔrelAΔspoTlpp3, W3110ΔrelAspoT1Δlpp und W3110ΔrelAspoT1lpp3 und mit dem Plasmid pCGT mittels der CaCl₂-Methode transformiert. Die Selektion auf plasmidhaltige Zellen erfolgte mittels Ampicillin (100 mg/l).

Das Plasmid pCGT enthält neben dem Gen für die Resistenz gegen Ampicillin u.a. auch das Strukturgen der CGTase einschließlich der nativen CGTase-Signalsequenz. Die Expression des CGTase-Gens steht unter der Kontrolle des tac-Promotors. Das Plasmid pCGT ist in Beispiel 4 von US2008254511 beschrieben.

Die CGTase-Produktion erfolgte in 10 L Rührkesselfermentern mit den Stämmen W3110ΔrelA/pCGT, W3110spoT1/pCGT, W3110ΔrelAΔspoT/pCGT, W3110ΔrelAspoT1/pCGT, W3110ΔrelAΔspoTΔlpp/pCGT, W3110ΔrelAΔspoTlpp3/pCGT, W3110ΔrelAspoT1Δlpp/pCGT und W3110ΔrelAspoT1lpp3/pCGT.

Der mit 6 1 des Fermentationsmediums FM4 (1,5 g/l KH₂PO₄; 5 g/l (NH₄)₂SO₄; 0,5 g/l MgSO₄ x 7 H₂O; 0,15 g/l CaCl₂ x 2 H₂O, 0,075 g/l FeSO₄ x 7 H₂O; 1 g/l Na₃Citrat x 2 H₂O; 0,5 g/l NaCl; 1 ml/l Spurenelementlösung (0,15 g/l Na₂MoO₄ x 2 H₂O; 2,5 g/l Na₃BO₃; 0,7 g/l CoCl₂ x 6 H₂O; 0,25 g/l CuSO₄ x 5 H₂O; 1,6 g/l MnCl₂ x 4 H₂O; 0,3 g/l ZnSO₄ x 7 H₂O); 5 mg/l Vitamin B₁; 3 g/l Phyton; 1,5 g/l Hefeextrakt; 10 g/l Glukose; 100 mg/l Ampicillin) befüllte Fermenter wurde im Verhältnis 1:10 mit einer Vorkultur, die übernacht im gleichen Medium kultiviert wurde, angeimpft. Während der Fermentation wurde eine Temperatur von 30 °C eingestellt und der pH-Wert durch Zudosierung von NH₄OH bzw. H₃PO₄ bei einem Wert von 7,0 konstant gehalten. Glukose wurde über die Fermentation hinweg zudosiert, wobei eine maximale Glukose-Konzentration im Fermentationsmedium von < 10 g/l angestrebt wurde. Die Induktion der Expression erfolgte durch Zugabe von Isopropyl-β-D-thiogalactopyranosid (IPTG) ad 0,1 mM am Ende der logarithmischen Wachstumsphase.

Nach 72 h Fermentation wurden Proben entnommen, die Zellen durch Zentrifugation vom Fermentationsmedium abgetrennt und der CGTase-Gehalt im Fermentationsüberstand durch folgenden Aktivitätstest bestimmt:
Testpuffer: 5 mM Tris-HCl-Puffer > pH 6,5, 5 mM CaSO₄ . 2 H₂O Substrat: 10 %ige Noreduxlösung in Test-Puffer (pH 6,5) Testansatz: 1 ml Substratlösung + 1 ml abzentrifugierter und
gegebenenfalls verdünnter Kulturüberstand (5 min, 12 000 rpm) + 3 ml Methanol
Reaktionstemperatur: 40°C

Enzymtest:
◇ Vortemperieren der Lösungen (ca. 5 min bei 40°C)
◇ Zugabe der Enzymlösung zur Substratlösung; rasches Mischen (Whirl-Mixer)
◇ Inkubation für 3 min bei 40°C
◇ Stoppen der Enzymreaktion durch Methanol-Zugabe; rasches Mischen (Whirl-Mixer)
◇ Abkühlen des Ansatzes auf Eis (ca. 5 min)
◇ Abzentrifugieren (5 min, 12 000 rpm) und Abpipettieren des klaren Überstandes
◇ Analyse des produzierten CDs mittels HPLC
Enzymaktivität: A = G*V1*V2/(t*MG) (Units/ml)
A = Aktivität
G = Gehalt an CD in mg/l = Testansatz: Flächeneinheiten x 10⁴ / Standardlösung (10mg/ml)/ Flächeneinheiten
V1 = Verdünnungsfaktor im Testansatz (bei Durchführung nach o.g. Angaben: V1 = 5)
V2 = Verdünnungsfaktor des Kulturüberstands vor dem Einsatz im Test; wenn unverdünnt: V2 = 1
t = Reaktionszeit in min
MG = Molekulargewicht in g/mol (CD = 973 g/mol)
1 Unit = 1 Mol Produkt / min
Aus der auf diese Weise bestimmten CGTase-Aktivität lässt sich die Menge der im Fermentationsüberstand vorhandenen CGTase berechnen. Dabei entsprechen 150 U/ml CGTase-Aktivität etwa 1 g/l CGTase-Protein.
Tabelle 1 zeigt die jeweils erzielten Cyclodextrin-Glycosyltransferase-Ausbeuten.

**Tabelle 1: Cyclodextrin-Glycosyltransferase-Ausbeuten im Fermentationsüberstand nach 72 Stunden Fermentation**

| Stamm | CGTase (U/ml) | CGTase (g/l) |
|---|---|---|
| W3110ΔrelA/pCGT | 220 | 1,5 |
| W3110spoT1/pCGT | 260 | 1,7 |
| W3110ΔrelAΔspoT/pCGT | 340 | 2,3 |
| W3110ΔrelAspoT1/pCGT | 360 | 2,4 |
| W3110ΔrelAΔspoTΔlpp/pCGT | 530 | 3,5 |
| W3110ΔrelAspoT1Δlpp/pCGT | 540 | 3,6 |
| W3110ΔrelAΔspoTlpp3/pCGT | 530 | 3,5 |
| W3110ΔrelAspoT1lpp3/pCGT | 550 | 3,7 |

### Beispiel 6: Fermentative Produktion eines Hirudin-Derivats im 10 1-Maßstab mit Stämmen, die eine attenuierte PSII-Aktivität aufweisen

In diesem Beispiel wird die fermentative Herstellung eines Hirudin-Derivats mit der N-terminalen Aminosäure-Sequenz Ala-Thr-Tyr-Thr-Asp beschrieben. Für die Produktion des Hirudin-Derivats wurden die Stämme W3110ΔrelA, W3110spoT1, W3110ΔrelAΔspoT, W3110ΔrelAspoT1, W3110ΔrelAΔspoTΔlpp, W3110ΔrelAΔspoTlpp3, W3110ΔrelAspoT1Δlpp und W3110ΔrelAspoT1lpp3 zunächst jeweils mit dem Plasmid pCMT203 mittels der CaCl₂-Methode transformiert. Die Selektion auf plasmidhaltige Zellen erfolgte mittels Ampicillin (100 mg/l).

Das Plasmid pCMT203 enthält neben dem Gen für die Resistenz gegen Ampicillin u.a. auch das Strukturgen des Hirudin-Derivats, welches *in frame* an das 3'-Ende einer CGTase-Signalsequenz anfusioniert ist. Die Expression der CGTase-Signalsequenz-Hirudin-Fusion steht unter der Kontrolle des tac-Promotors. Das Plasmid pCMT203 ist in EP0448093 beschrieben.

Die Produktion des Hirudin-Derivats im 10 l-Maßstab erfolgte analog dem in Beispiel 5 beschriebenen Verfahren mit den Stämmen W3110ΔrelA/pCMT203, W3110spoT1/pCMT203,

W3110ΔrelAΔspoT/pCMT203, W3110ΔrelAspoT1/pCMT203, W3110ΔrelAΔspoTΔlpp/pCMT203, W3110ΔrelAΔSpoTlpp3/pCMT203, W3110ΔrelAspoT1Δlpp/pCMT203 und W3110ΔrelAspoT1lpp3/pCMT203. Nach 72 h Fermentation wurden Proben entnommen, anschließend die Zellen durch Zentrifugation vom Fermentationsmedium abgetrennt und der Hirudin-Gehalt im Fermentationsüberstand mittels eines Thrombin-Inaktiverungstests wie in Chang (1991, J. Biol. Chem. 266, 10839-43) beschrieben bestimmt. Dabei entsprechen 16000 Antithrombin(AT)-Units/ml Hirudin-Aktivität etwa 1 g/l Hirudin-Protein.

Tabelle 2 zeigt die jeweils erzielten Hirudin-Ausbeuten im Fermentationsüberstand.

**Tabelle 2: Hirudin-Ausbeute (in AT-U/ml und g/l) im Fermentationsüberstand nach 72 h Fermentation.**

| Stamm | Hirudin (AT- | Hirudin |
|---|---|---|
| | U/ml) | (g/l) |
| W3110ΔrelA/pCMT203 | 1000 | 0,06 |
| W3110spoT1/pCMT203 | 1400 | 0,09 |
| W3110ΔrelAΔspoT/pCMT203 | 2500 | 0,16 |
| W3110ΔrelAspoT1/pCMT203 | 2800 | 0,18 |
| W3110ΔrelAΔspoTΔlpp/pCMT203 | 52000 | 3,25 |
| W3110ΔrelASpoT1Δlpp/pCMT203 | 54000 | 3,38 |
| W3110ΔrelAΔspoTlpp3/pCMT203 | 53000 | 3,31 |
| W3110ΔrelAspoT1lpp3/pCMT203 | 56000 | 3,50 |

### Beispiel 7: Fermentative Produktion von Interferonα2b im 10 l-Maßstab mit Stämmen, die eine attenuierte PSII-Aktivität aufweisen

Für die Herstellung von Interferonα2b wurden die Stämme W3110ΔrelA, W3110spoT1, W3110ΔrelAΔspoT, W3110ΔrelAspoT1, W3110ΔrelAΔspoTΔlpp, W3110ΔrelAΔspoTlpp3, W3110ΔrelAspoT1Δlpp und W3110ΔrelAspoT1lpp3 jeweils mit dem Plasmid pIFN nach der CaCl₂-Methode transformiert. Die Selektion auf plasmidhaltige Zellen erfolgte mittels Ampicillin (100 mg/l).

Das Plasmid pIFN enthält neben dem Gen für die Resistenz gegen Ampicillin u.a. auch das Strukturgen von Interferonα2b, welches *in frame* an das 3'-Ende einer CGTase-Signalsequenz anfusioniert ist. Die Expression der CGTase-Signalsequenz-Interferonα2b-Fusion steht unter der Kontrolle des tac-Promotors. Das Plasmid pIFN ist in Beispiel 6 von US2008254511 beschrieben.

Die Produktion von Interferonα2b im 10 l-Maßstab erfolgte analog dem in Beispiel 5 beschriebenen Verfahren mit den Stämmen W3110ΔrelA/pIFN, W3110spoT1/pIFN, W3110ΔrelAΔspoT/pIFN, W3110ΔrelAspoT1/pIFN, W3110ΔrelAΔspoTΔlpp/pIFN, W3110ΔrelAΔspoTlpp3/pIFN, W3110ΔrelAspoT1Δlpp/pIFN und W3110ΔrelAspoT1lpp3/pIFN. Nach 72 h Fermentation wurden Proben entnommen, anschließend die Zellen durch Zentrifugation vom Fermentationsmedium abgetrennt und der Interferonα2b-Gehalt im Fermentationsüberstand bestimmt.

Dazu wurden die Proteine im Fermentationsüberstand elektrophoretisch in einem SDS-Polyacrylamidgel aufgetrennt und mittels Detektion im Immunoblot mit Anti-Interferon-spezifischen Antikörpern wie folgt quantifiziert:
1 µl Überstand wurde mit Probenpuffer versetzt (2x Tris SDS - Sample Buffer (Invitrogen Cat.No. LC2676): 0.125 M Tris.HCl, pH 6.8, 4% w/v SDS, 20% v/v Glycerin, 0.005% v/v Bromphenolblau, 5% beta-Mercaptoethanol). Außerdem wurden definierte Mengen von Interferonα2b als Standard mit aufgetragen. Denaturieren der Proteine erfolgte durch Erhitzen auf 100°C für 5 min, Abkühlen für 2 min auf Eis und abzentrifugieren.

Die Proteine wurden durch Elektrophorese in einem 12% NuPAGE^{®} Bis-Tris-Gel (Invitrogen Cat.No. NP0341) mit 1x MES-haltigem Running Buffer (Invitrogen Cat.No. NP0002) aufgetrennt (Elektrophorese-Parameter: 40 min bei 200 V).

Detektion und Quantifizierung über Immunoblot wurde nach folgender Vorschrift durchgeführt:

### Transfer im Naßblot-Verfahren:

Modul: Amersham: Hoefer TE 22 Mini Tank Transfer Unit, Code Number: 80-6204-26

Membran: Nitrocellulose-Membran (Schleicher&Schuell, BA 85, Cellulosenitrat (E), 0,45 µm Porengröße)

Whatman-Filter und Nitrocellulose-Membran in passende Größe schneiden und mit Schaumstoffstücken (Schwämmen) in Transferpuffer (Invitrogen Cat.No. LC3675) luftblasenfrei tränken. Aufbau des Sandwiches: schwarzes Gitter, Verbindung mit der Kathode, 2 Schwämme, je 3 mm dick, Whatman-Papier, SDS-Polyacrylamidgel, NC-Membran, Whatman, 1 Schwamm, 6 mm dick, weißes Gitter, Verbindung mit der Anode.

Transferbedingungen: I = 200mA constant current, U = unbegrenzt, Laufzeit 60 min

### Prehybridisierung

Inkubation der Membran in 25 ml Prehybridisierungspuffer 30 min bei RT schwenken

### Hybridisierung 1. Antikörper

Inkubation der Membran in 25 ml Prehybridisierungspuffer + 0,15 µg/ml (-> 3,75 µg) Anti-human-IFN-alpha Antibody (Pepro Tech EC, über Biozol Cat. No.: 500-P32A) 90 min oder über Nacht bei RT schwenken

### Waschen

10 Sekunden mit 1 x PBS schwenken, RT, Puffer abgießen 2 x 15 min mit 1x PBS schwenken, RT, Puffer abgießen

### Hybridisierung 2. Antikörper

Inkubation der Membran in 25 ml Prehybridisierungspuffer + 25 µl (1:1000) Goat Anti-Rabbit IgG Horseradish Peroxidase Conjugate (HRP) (Southern Biotech, über Biozol Cat. No.: 4050-05) 60 min bei RT schwenken

### Waschen

10 Sekunden mit 1 x PBS schwenken, RT, Puffer abgießen 2 x 15 min mit 1 x PBS schwenken, RT, Puffer abgießen

### Detektion über Chemilumineszenz

Lumi-Light Western Blotting Substrate (Roche, Cat. No.: 2015200) vorbereiten: Lumi-Light Luminol / Enhancer Solution und Lumi-Light Stable Peroxide Solution im Verhältnis 1:1 mischen: 3ml / NC-Membran.

Blot 5 min bei RT mit Lumi-Light Western Blotting Substrate inkubieren, Überschuss ablaufen lassen, Membran mit Frischhaltefolie bedecken und sofort einen X-Ray Film (Kodak, X-OMAT) auflegen, 2 min exponieren, entwickeln und fixieren. Bei schwachen Signalen wird die Exposition über einen längeren Zeitraum wiederholt.

### Puffer

### Prehybridisierungspuffer: 5 % Magermilchpulver in 1x PBS

10x PBS: 100 mM NaH₂PO₄, 1,5 M NaCl, pH 7,5 mit NaOH, 0,5 % Triton 100

1x PBS: 10x PBS mit vollentsalztem Wasser 1:10 verdünnen

### Quantifizierung

Eine quantitative Auswertung erfolgte nach Einscannen des Immunoblots mit Biorad GS-800 Calibrated Densitometer mittels der Quantity One 1-D-Analysis Software (Biorad) durch Vergleich mit dem aufgetragenen Standard.

In Tabelle 3 sind die auf diese Weise bestimmten Interferonα2b-Ausbeuten im Fermentationsüberstand dargestellt.

**Tabelle 3:** Interferonα2b-Ausbeuten im Fermentationsüberstand nach 72 h Fermentation.

| Stamm | Interferonα2b |
|---|---|
| | (mg/l) |
| W3110ΔrelA/pIFN | 10 |
| W3110spoT1/pIFN | 20 |
| W3110ΔrelAΔspoT/pIFN | 40 |
| W3110ΔrelAspoT1/pIFN | 50 |
| W3110ΔrelAΔspoTΔlpp/pIFN | 580 |
| W3110ΔrelAspoT1Δlpp/pIFN | 600 |
| W3110ΔrelAΔspoTlpp3/pIFN | 590 |
| W3110ΔrelAspoT1lpp3/pIFN | 610 |

### Beispiel 8: Fermentative Produktion von Fab-Antikörperfragmenten im 10 l-Maßstab mit Stämmen, die eine attenuierte PSII-Aktivität aufweisen

Das vorliegende Beispiel beschreibt die Produktion eines Fab-Fragments des gut charakterisierten Anti-Lysozym-Antikörpers D1.3.

Für die Herstellung des Anti-Lysozym-Fab-Fragments wurden die Stämme W3110ΔrelA, W3110spoT1, W3110ΔrelAΔspoT, W3110ΔrelAspoT1, W3110ΔrelAΔspoTΔlpp, W3110ΔrelAΔspoTlpp3, W3110ΔrelAspoT1Δlpp und W3110ΔrelAspoT1lpp3 jeweils mit dem Plasmid pFab-Anti-Lysozym mit der CaCl₂-Methode transformiert. Die Selektion auf plasmidhaltige Zellen erfolgte mittels Ampicillin (100 mg/l).

Das Plasmid pFab-Anti-Lysozym enthält neben dem Gen für die Resistenz gegen Ampicillin u.a. auch die Strukturgene für die HC und die LC des Fab-Fragments in Form eines Operons. Dabei ist die HC *in frame* an das 3'-Ende der ompA-Signalsequenz (ompA^{ss}) und die LC *in frame* an das 3'-Ende einer CGTase-Signalsequenz (CGT^{ss}) anfusioniert. Die Expression des ompA^{ss}-HC-CGT^{ss}-LC-Operons steht unter der Kontrolle des tac-Promotors. Das Plasmid pFab-Anti-Lysozym ist in Beispiel 7 von US2008254511 beschrieben.

Die Produktion des Anti-Lysozym-Fab-Fragments im 10 l-Maßstab erfolgte analog dem in Beispiel 5 beschriebenen Verfahren mit den Stämmen W3110ΔrelA/pFab-Anti-Lysozym, W3110spoT1/pFab-Anti-Lysozym, W3110ΔrelAΔspoT/pFab-Anti-Lysozym, W3110ΔrelAspoT1/pFab-Anti-Lysozym, W3110ΔrelAΔspoTΔlpp/pFab-Anti-Lysozym, W3110ΔrelAΔspoTlpp3/pFab-Anti-Lysozym, W3110ΔrelAspoT1Δlpp/pFab-Anti-Lysozym und W3110ΔrelAspoT1lpp3/pFab-Anti-Lysozym. Nach 72 h Fermentation wurden Proben entnommen und anschließend die Zellen durch Zentrifugation vom Fermentationsmedium abgetrennt.

Die Reinigung des Anti-Lysozym-Fab-Fragments aus den Fermentationsüberstanden erfolgte mittels Affinitätschromatographie wie in Skerra (1994, Gene 141, 79-84) beschrieben.

Die Quantifizierung sowie die Bestimmung der Aktivität des gereinigten Anti-Lysozym-Fab-Fragments erfolgte über einen ELISA-Test mit Lysozym als Antigen (Skerra, 1994, Gene 141, 79-84).

In Tabelle 4 sind die Ausbeuten an funktionellem Anti-Lysozym-Fab-Fragment aufgelistet, die aus jeweils 20 ml Fermentationsüberstand nach 72 h Fermentation isoliert werden konnten.

**Tabelle 4**: Anti-Lysozym-Fab-Fragment-Ausbeuten im Fermentationsüberstand nach 72 h Fermentation.

**Beispiel 9: Fermentative Produktion von Volllängen-Antikörpern im 10 l-Maßstab mit Stämmen, die eine attenuierte PSII-Aktivität aufweisen**

| Stamm | aus 20 ml Überstand gereinigtes Anti-Lysozym-Fab-Fragment [mg] | Anti-Lysozym-Fab-Fragment-Ausbeute Fragment-Ausbeute [g/l] im Fermentationsüberstand (hochgerechnet) |
|---|---|---|
| W3110ΔrelA/ pFab-Anti-Lysozym | 0,5 | 0,03 |
| W3110spoT1/ pFab-Anti-Lysozym | 1 | 0,05 |
| W3110ΔrelAΔspoT/ pFab-Anti-Lysozym | 4 | 0,20 |
| W3110ΔrelAspoT1/ pFab-Anti-Lysozym | | 3 0,15 |
| W3110ΔrelAΔspoTΔlpp/ pFab-Anti-Lysozym | 32 | 1,60 |
| W3110ΔrelAspoT1Δlpp/ pFab-Anti-Lysozym | 36 | 1,80 |
| W3110ΔrelAΔspoTlpp3/ pFab-Anti-Lysozym | 34 | 1,7 |
| W3110ΔrelAspoT1lpp3/ pFab-Anti-Lysozym | 37 | 1,85 |

Das vorliegende Beispiel beschreibt die Produktion des Anti-Tissue factor (αTF) IgG1-Antikörpers.

Für die Herstellung des Anti-αTF-Antikörpers wurden die Stämme W3110ΔrelA, W3110spoT1, W3110ΔrelAΔspoT, W3110ΔrelAspoT1, W3110ΔrelAΔspoTΔlpp, W3110ΔrelAΔspoTlpp3, W3110ΔrelAspoT1Δlpp und W3110ΔrelAspoT1lpp3 jeweils mit dem Plasmid pAK-Anti-TF mit der CaCl₂-Methode transformiert. Die Selektion auf plasmidhaltige Zellen erfolgte mittels Ampicillin (100 mg/l).

Das Plasmid pAK-Anti-TF enthält neben dem Gen für die Resistenz gegen Ampicillin u.a. auch die Strukturgene für die HC und die LC des Anti-αTF-Antikörpers in Form eines Operons. Dabei ist die HC *in frame* an das 3'-Ende der ompA-Signalsequenz (ompA^{ss}) und die LC *in frame* an das 3'-Ende einer CGTase-Signalsequenz (CGT^{ss}) anfusioniert. Die Expression des ompA^{ss}-HC-CGT^{ss}-LC-Operons steht unter der Kontrolle des tac-Promotors. Das Plasmid pAK-Anti-TF ist in Beispiel 8 von US2008254511 beschrieben.

Die Produktion des Anti-αTF-Antikörpers im 10 l-Maßstab erfolgte analog dem in Beispiel 5 beschriebenen Verfahren mit den Stämmen W3110ΔrelA/pAK-Anti-TF, W3110spoT1/pAK-Anti-TF, W3110ΔrelAΔspoT/pAK-Anti-TF, W3110ΔrelAspoT1/pAK-Anti-TF, W3110ΔrelAΔspoTΔlpp/pAK-Anti-TF, W3110ΔrelAΔspoTlpp3/pAK-Anti-TF, W3110ΔrelAspoT1Δlpp/pAK-Anti-TF und W3110ΔrelAspoT1lpp3/pAK-Anti-TF. Nach 72 h Fermentation wurden Proben entnommen und anschließend die Zellen durch Zentrifugation vom Fermentationsmedium abgetrennt.

Die Quantifizierung des in das Fermentationsmedium sekretierten Anti-αTF-Antikörpers erfolgte über eine Aktivitäts-Bestimmung mit einem ELISA-Test mit löslichem Tissue factor als Antigen (Coating) und einem Peroxidase-konjugierten goat Anti-human F(ab')₂-Fragment als sekundärem Antikörper wie in Simmons et al. (2002, J. Immunol. Methods 263, 133-47) beschrieben.

In Tabelle 5 sind die auf diese Weise bestimmten Ausbeuten an funktionellem Anti-αTF-Antikörper aufgelistet.

**Tabelle 5**: Anti-αTF-Antikörper-Ausbeuten im Fermentationsüberstand nach 72 h Fermentation.

| Stamm | Anti-αTF-Antikörper [mg/l] |
|---|---|
| W3110ΔrelA/ pAK-Anti-TF | 8 |
| W3110spoT1/ pAK-Anti-TF | 11 |
| W3110ΔrelAΔspoT/ pAK-Anti-TF | 21 |
| W3110ΔrelAspoT1/ pAK-Anti-TF | 18 |
| W3110ΔrelAΔspoTΔlpp/ pAK-Anti-TF | 680 |
| W3110ΔrelAspoT1Δlpp/ pAK-Anti-TF | 660 |
| W3110ΔrelAΔspoTlpp3/ pAK-Anti-TF | 670 |
| W3110ΔrelAspoT1lpp3/ pAK-Anti-TF | 670 |

## Patentansprüche

1. Verfahren zur Herstellung eines heterologen Proteins, bei dem ein E. coli-Stamm in einem Fermentationsmedium kultiviert wird, wobei der E. coli-Stamm ein Gen kodierend für das heterologe Protein, funktionell verknüpft mit einer Signalsequenz kodierend für ein Signalpeptid, enthält und er das heterologe Protein in das Fermentationsmedium ausscheidet und das heterologe Protein vom Fermentationsmedium abgetrennt wird, **dadurch gekennzeichnet, dass** der E. coli-Stamm eine attenuierte (p)ppGpp-Synthetase II-Aktivität (PSII-Aktivität) aufweist.

2. Verfahren gemäß Anspruch 1, **dadurch gekennzeichnet, dass** der E. coli-Stamm maximal 50%, bevorzugt maximal 20% der PSII-Aktivität des Stammes vor Modifikation seiner PSII-Aktivität aufweist.

3. Verfahren gemäß Anspruch 1 oder 2, **dadurch gekennzeichnet, dass** der E. coli-Stamm eine spoT1-Mutation oder eine spoT-Mutation, die zu einer attenuierten PSII-Aktivität führt, aufweist.

4. Verfahren gemäß Anspruch 1, 2 oder 3, **dadurch gekennzeichnet, dass** der E. coli-Stamm eine spoT-Mutation, die den vollständigen Verlust der PSII-Aktivität bewirkt, aufweist.

5. Verfahren gemäß einem der Ansprüche 1 bis 4, **dadurch gekennzeichnet, dass** der E. coli-Stamm zusätzlich eine Mutation im relA-Gen aufweist, welche zu einem relaxierten Phänotyp führt.

6. Verfahren gemäß Anspruch 5, **dadurch gekennzeichnet, dass** die relA-Mutation einen vollständigen Verlust der PSI-Aktivität bewirkt.

7. Verfahren gemäß einem der Ansprüche 1 bis 6, **dadurch gekennzeichnet, dass** der E. coli-Stamm eine "leaky"-Mutation in einem Gen ausgewählt aus der Gruppe der omp-Gene, tol-Gene, excD-Gen, excC-Gen, lpp-Gen, pal-Gen, env-Gene und leaky-Gene aufweist.

8. Verfahren gemäß Anspruch 7, **dadurch gekennzeichnet, dass** die leaky"-Mutation eine Mutation im lpp-Gen, besonders bevorzugt eine Mutation ausgewählt aus der Gruppe lpp1-Mutation, lpp3-Mutation und lpp-Deletionsmutation ist.

9. Verfahren gemäß einem der Ansprüche 1 bis 8, **dadurch gekennzeichnet, dass** das heterologe Protein ein eukaryontisches Protein ist.

10. Verfahren gemäß einem der Ansprüche 1 bis 9, **dadurch gekennzeichnet, dass** die Signalsequenz die Signalsequenz des phoA- oder des ompA-Gens von E. coli oder die Signalsequenz für eine Cyclodextrin-Glycosyltransferase (CGTase) aus Klebsiella pneumoniae M5a1, oder die von dieser Signalsequenz abgeleitete Sequenz SEQ ID No. 5 ist.

## Claims

1. Process for producing a heterologous protein, which comprises culturing an *E. coli* strain in a fermentation medium, said *E. coli* strain harboring a gene which codes for said heterologous protein and is functionally linked to a signal sequence coding for a signal peptide, and releasing said heterologous protein into the fermentation medium, and said heterologous protein being removed from said fermentation medium, **characterized in that** the *E. coli* strain has an attenuated (p)ppGpp-synthetase II activity (PSII activity).

2. Process according to Claim 1, **characterized in that** the *E. coli* strain has no more than 50%, preferably no more than 20%, of the PSII activity of said strain prior to modification of its PSII activity.

3. Process according to Claim 1 or 2, **characterized in that** the *E*. *coli* strain has a spoT1 mutation or a spoT mutation, resulting in attenuated PSII activity.

4. Process according to Claim 1, 2 or 3, **characterized in that** the *E. coli* strain has a spoT mutation which causes the complete loss of PSII activity.

5. Process according to any of Claims 1 to 4, **characterized in that** the *E. coli* strain additionally has a mutation in the relA gene, which mutation results in a relaxed phenotype.

6. Process according to Claim 5, **characterized in that** the relA mutation causes a complete loss of PSI activity.

7. Process according to any of Claims 1 to 6, **characterized in that** the *E*. *coli* strain has a "leaky" mutation in a gene selected from the group consisting of omp genes, tol genes, excD gene, excC gene, lpp gene, pal gene, env genes, and leaky genes.

8. Process according to Claim 7, **characterized in that** the "leaky" mutation is a mutation in the lpp gene, particularly preferably a mutation selected from the group consisting of lpp1 mutation, lpp3 mutation and lpp deletion mutation.

9. Process according to any of Claims 1 to 8, **characterized in that** the heterologous protein is a eukaryotic protein.

10. Process according to one of Claims 1 to 9, **characterized in that** the signal sequence is the signal sequence of the *E. coli* phoA or ompA gene, or the signal sequence for a *Klebsiella pneumoniae* M5a1 cyclodextrin glycosyltransferase (CGTase), or the sequence derived from this signal sequence, SEQ ID No. 5.

## Revendications

1. Procédé de préparation d'une protéine hétérologue, dans lequel une souche de E. coli est cultivée dans un milieu de fermentation, la souche de E. coli contenant un gène codant pour la protéine hétérologue fonctionnellement lié à une séquence signal codant pour un peptide signal, et elle excrète la protéine hétérologue dans le milieu de fermentation, et la protéine hétérologue est séparée du milieu de fermentation, **caractérisé en ce que** la souche de E. coli présente une activité atténuée de (p)ppGpp-synthétase II (activité de PSII).

2. Procédé selon la revendication 1, **caractérisé en ce que** la souche de E. coli présente une activité de PSII au maximum de 50 %, de préférence au maximum de 20 %, de celle de la souche avant modification de son activité de PSII.

3. Procédé selon la revendication 1 ou 2, **caractérisé en ce que** la souche de E. coli présente une mutation spoT1 ou une mutation spoT, qui conduit à une activité atténuée de PSII.

4. Procédé selon la revendication 1, 2 ou 3, **caractérisé en ce que** la souche de E. coli présente une mutation spoT, qui provoque une perte complète de l'activité de PSII.

5. Procédé selon l'une des revendications 1 à 4, **caractérisé en ce que** la souche de E. coli présente en outre une mutation dans le gène relA, qui conduit à un phénotype relaxé.

6. Procédé selon la revendication 5, **caractérisé en ce que** la mutation relA provoque une perte complète de l'activité de PSI.

7. Procédé selon l'une des revendications 1 à 6, **caractérisé en ce que** la souche de E. coli présente une mutation "fuyante" dans un gène choisi dans le groupe des gènes omp, des gènes tol, du gène excD, du gène excC, du gène lpp, du gène pal, des gènes env et des gènes fuyants.

8. Procédé selon la revendication 7, **caractérisé en ce que** la mutation "fuyante" est une mutation dans le gène lpp, d'une manière particulièrement préférée une mutation choisie dans le groupe consistant en la mutation lpp1, la mutation lpp3 et la mutation par délétion lpp.

9. Procédé selon l'une des revendications 1 à 8, **caractérisé en ce que** la protéine hétérologue est une protéine eucaryote.

10. Procédé selon l'une des revendications 1 à 9, **caractérisé en ce que** la séquence signal est la séquence signal du gène phoA ou du gène ompA de E. coli, ou la séquence signal pour une cyclodextrine-glycosyltransférase (CGTase) de Klebsiella pneumoniae M5a1, ou la séquence SEQ ID N° 5 qui dérive de cette séquence signal.


### SEQUENCE LISTING

<110> Wacker Chemie AG
<120> Verfahren zur fermentativen Herstellung von heterologen Proteinen mittels Escherichia coli
<130> Co10813
<140>
   <141>
<160> 17
<270> PatentIn Ver. 2.0
<210> 1
   <211> 2109
   <212> DNA
   <213> Escherichia coli
<220>
   <221> gene
   <222> (1)..(2109)
   <223> spoT-Gen (Wildtyp)
<300>
<301> Sarubbi, Edoardo
   <302> Characterization of the spoT gene of Escherichia coli.
   <303> J. Biol. Chem.
   <304> 264
   <305> 25
   <306> 15074-15082
   <307> 1989
<400> 1
   ttgtatctgt ttgaaagcct gaatcaactg attcaaacct acctgccgga agaccaaatc 60
   aagcgtctgc ggcaggcgta tctcgttgca cgtgatgctc acgaggggca aacacgttca 120
   agcggtgaac cctatatcac gcacccggta gcggttgcct gcattctggc cgagatgaaa 180
   ctcgactatg aaacgctgat ggcggcgctg ctgcatgacg tgattgaaga tactcccgcc 240
   acctaccagg atatggaaca gctttttggt aaaagcgtcg ccgagctggt agagggggtg 300
   tcgaaacttg ataaactcaa gttccgcgat aagaaagagg cgcaggccga aaactttcgc 360
   aagatgatta tggcgatggt gcaggatatc cgcgtcatcc tcatcaaact tgccgaccgt 420
   acccacaaca tgcgcacgct gggctcactt cgcccggaca aacgtcgccg catcgcccgt 480
   gaaactctcg aaatttatag cccgctggcg caccgtttag gtatccacca cattaaaacc 540
   gaactcgaag agctgggttt tgaggcgctg tatcccaacc gttatcgcgt aatcaaagaa 600
   gtggtgaaag ccgcgcgcgg caaccgtaaa gagatgatcc agaagattct ttctgaaatc 660
   gaagggcgtt tgcaggaagc gggaataccg tgccgcgtca gtggtcgcga gaagcatctt 720
   tattcgattt actgcaaaat ggtgctcaaa gagcagcgtt ttcactcgat catggacatc 780
   tacgctttcc gcgtgatcgt caatgattct gacacctgtt atcgcgtgct gggccagatg 840
   cacagcctgt acaagccgcg tccgggccgc gtgaaagact atatcgccat tccaaaagcg 900
   aacggctatc agtctttgca cacctcgatg atcggcccgc acggtgtgcc ggttgaggtc 960
   cagatccgta ccgaagatat ggaccagatg gcggagatgg gtgttgccgc gcactgggct 1020
   tataaagagc acggcgaaac cagtactacc gcacaaatcc gcgcccagcg ctggatgcaa 1080
   agcctgctgg agctgcaaca gagcgccggt agttcgtttg aatttatcga gagcgttaaa 1140
   tccgatctct tcccggatga gatttacgtt ttcacaccgg aagggcgcat tgtcgagctg 1200
   cctgccggtg caacgcccgt cgacttcgct tatgcagtgc ataccgatat cggtcatgcc 1260
   tgcgtgggcg cacgcgttga ccgccagcct tacccgctgt cgcagccgct taccagcggt 1320
   caaaccgttg aaatcattac cgctccgggc gctcgcccga atgccgcttg gctgaacttt 1380
   gtcgttagct cgaaagcgcg cgccaaaatt cgtcagttgc tgaaaaacct caagcgtgat 1440
   gattctgtaa gcctgggccg tcgtctgctc aaccatgctt tgggtggtag ccgtaagctg 1500
   aatgaaatcc cgcaggaaaa tattcagcgc gagctggatc gcatgaagct ggcaacgctt 1560
   gacgatctgc tggcagaaat cggacttggt aacgcaatga gcgtggtggt cgcgaaaaat 1620
   ctgcaacatg gggacgcctc cattccaccg gcaacccaaa gccacggaca tctgcccatt 1680
   aaaggtgccg atggcgtgct gatcaccttt gcgaaatgct gccgccctat tcctggcgac 1740
   ccgattatcg cccacgtcag ccccggtaaa ggtctggtga tccaccatga atcctgccgt 1800
   aatatccgtg gctaccagaa agagccagag aagtttatgg ctgtggaatg ggataaagag 1860
   acggcgcagg agttcatcac cgaaatcaag gtggagatgt tcaatcatca gggtgcgctg 1920
   gcaaacctga cggcggcaat taacaccacg acttcgaata ttcaaagttt gaatacggaa 1980
   gagaaagatg gtcgcgtcta cagcgccttt attcgtctga ccgctcgtga ccgtgtgcat 2040
   ctggcgaata tcatgcgcaa aatccgcgtg atgccagacg tgattaaagt cacccgaaac 2100
   cgaaattaa                                                         2109
<210> 2

   <211> 702
   <212> PRT
   <213> Escherichia coli
<220>
   <221> PEPTIDE
   <222> (1)..(702)
   <223> SpoT-Protein (Wildtyp)
<400> 2
   Leu Tyr Leu Phe Glu Ser Leu Asn Gln Leu Ile Gln Thr Tyr Leu Pro
   1               5                   10                  15
   Glu Asp Gln Ile Lys Arg Leu Arg Gln Ala Tyr Leu Val Ala Arg Asp
               20                  25                  30
   Ala His Glu Gly Gln Thr Arg Ser Ser Gly Glu Pro Tyr Ile Thr His
   35 40 45
   Pro Val Ala Val Ala Cys Ile Leu Ala Glu Met Lys Leu Asp Tyr Glu
   50 55 60
   Thr Leu Met Ala Ala Leu Leu His Asp Val Ile Glu Asp Thr Pro Ala
   65 70 75 80
   Thr Tyr Gln Asp Met Glu Gln Leu Phe Gly Lys Ser Val Ala Glu Leu
   85 90 95
   Val Glu Gly Val Ser Lys Leu Asp Lys Leu Lys Phe Arg Asp Lys Lys
   100 105 110
   Glu Ala Gln Ala Glu Asn Phe Arg Lys Met Ile Met Ala Met Val Gln
   115 120 125
   Asp Ile Arg Val Ile Leu Ile Lys Leu Ala Asp Arg Thr His Asn Met
   130 135 140
   Arg Thr Leu Gly Ser Leu Arg Pro Asp Lys Arg Arg Arg Ile Ala Arg
   145 150 155 160
   Glu Thr Leu Glu Ile Tyr Ser Pro Leu Ala His Arg Leu Gly Ile His
   165 170 175
   His Ile Lys Thr Glu Leu Glu Glu Leu Gly Phe Glu Ala Leu Tyr Pro
   180 185 190
   Asn Arg Tyr Arg Val Ile Lys Glu Val Val Lys Ala Ala Arg Gly Asn
   195 200 205
   Arg Lys Glu Met Ile Gln Lys Ile Leu Ser Glu Ile Glu Gly Arg Leu
   210 215 220
   Gln Glu Ala Gly Ile Pro Cys Arg Val Ser Gly Arg Glu Lys His Leu
   225 230 235 240
   Tyr Ser Ile Tyr Cys Lys Met Val Leu Lys Glu Gln Arg Phe His Ser
   245 250 255
   Ile Met Asp Ile Tyr Ala Phe Arg Val Ile Val Asn Asp Ser Asp Thr
   260 265 270
   Cys Tyr Arg Val Leu Gly Gln Met His Ser Leu Tyr Lys Pro Arg Pro
   275 280 285
   Gly Arg Val Lys Asp Tyr Ile Ala Ile Pro Lys Ala Asn Gly Tyr Gln
   290 295 300
   Ser Leu His Thr Ser Met Ile Gly Pro His Gly Val Pro Val Glu Val
   305 310 315 320
   Gln Ile Arg Thr Glu Asp Met Asp Gln Met Ala Glu Met Gly Val Ala
   325 330 335
   Ala His Trp Ala Tyr Lys Glu His Gly Glu Thr Ser Thr Thr Ala Gln
   340 345 350
   Ile Arg Ala Gln Arg Trp Met Gln Ser Leu Leu Glu Leu Gln Gln Ser
   355 360 365
   Ala Gly Ser Ser Phe Glu Phe Ile Glu Ser Val Lys Ser Asp Leu Phe
   370 375 380
   Pro Asp Glu Ile Tyr Val Phe Thr Pro Glu Gly Arg Ile Val Glu Leu
   385 390 395 400
   Pro Ala Gly Ala Thr Pro Val Asp Phe Ala Tyr Ala Val His Thr Asp
   405 410 415
   Ile Gly His Ala Cys Val Gly Ala Arg Val Asp Arg Gln Pro Tyr Pro
   420 425 430
   Leu Ser Gln Pro Leu Thr Ser Gly Gln Thr Val Glu Ile Ile Thr Ala
   435 440 445
   Pro Gly Ala Arg Pro Asn Ala Ala Trp Leu Asn Phe Val Val Ser Ser
   450 455 460
   Lys Ala Arg Ala Lys Ile Arg Gln Leu Leu Lys Asn Leu Lys Arg Asp
   465 470 475 480
   Asp Ser Val Ser Leu Gly Arg Arg Leu Leu Asn His Ala Leu Gly Gly
   485 490 495
   Ser Arg Lys Leu Asn Glu Ile Pro Gln Glu Asn Ile Gln Arg Glu Leu
   500 505 510
   Asp Arg Met Lys Leu Ala Thr Leu Asp Asp Leu Leu Ala Glu Ile Gly
   515 520 525
   Leu Gly Asn Ala Met Ser Val Val Val Ala Lys Asn Leu Gln His Gly
   530 535 540
   Asp Ala Ser Ile Pro Pro Ala Thr Gln Ser His Gly His Leu Pro Ile
   545 550 555 560
   Lys Gly Ala Asp Gly Val Leu Ile Thr Phe Ala Lys Cys Cys Arg Pro
   565 570 575
   Ile Pro Gly Asp Pro Ile Ile Ala His Val Ser Pro Gly Lys Gly Leu
   580 585 590
   Val Ile His His Glu Ser Cys Arg Asn Ile Arg Gly Tyr Gln Lys Glu
   595 600 605
   Pro Glu Lys Phe Met Ala Val Glu Trp Asp Lys Glu Thr Ala Gln Glu
   610 615 620
   Phe Ile Thr Glu Ile Lys Val Glu Met Phe Asn His Gln Gly Ala Leu
   625 630 635 640
   Ala Asn Leu Thr Ala Ala Ile Asn Thr Thr Thr Ser Asn Ile Gln Ser
   645 650 655
   Leu Asn Thr Glu Glu Lys Asp Gly Arg Val Tyr Ser Ala Phe Ile Arg
   660 665 670
   Leu Thr Ala Arg Asp Arg Val His Leu Ala Asn Ile Met Arg Lys Ile
   675 680 685
   Arg Val Met Pro Asp Val Ile Lys Val Thr Arg Asn Arg Asn
   690 695 700
<210> 3
   <211> 704
   <212> PRT
   <213> Escherichia coli
<220>
   <221> PEPTIDE
   <222> (1) .. (704)
   <223> SpoT-Protein (SpoT1-Variante)
<400> 3
   Leu Tyr Leu Phe Glu Ser Leu Asn Gln Leu Ile Gln Thr Tyr Leu Pro
   1 5 10 15
   Glu Asp Gln Ile Lys Arg Leu Arg Gln Ala Tyr Leu Val Ala Arg Asp
   20 25 30
   Ala His Glu Gly Gln Thr Arg Ser Ser Gly Glu Pro Tyr Ile Thr His
   35 40 45
   Pro Val Ala Val Ala Cys Ile Leu Ala Glu Met Lys Leu Asp Tyr Glu
   50 55 60
   Thr Leu Met Ala Ala Leu Leu His Asp Val Ile Glu Asp Thr Pro Ala
   65 70 75 80
   Thr Tyr Gln Asp Gln Asp Met Glu Gln Leu Phe Gly Lys Ser Val Ala
   85 90 95
   Glu Leu Val Glu Gly Val Ser Lys Leu Asp Lys Leu Lys Phe Arg Asp
   100 105 110
   Lys Lys Glu Ala Gln Ala Glu Asn Phe Arg Lys Met Ile Met Ala Met
   115 120 125
   Val Gln Asp Ile Arg Val Ile Leu Ile Lys Leu Ala Asp Arg Thr His
   130 135 140
   Asn Met Arg Thr Leu Gly Ser Leu Arg Pro Asp Lys Arg Arg Arg Ile
   145 150 155 160
   Ala Arg Glu Thr Leu Glu Ile Tyr Ser Pro Leu Ala His Arg Leu Gly
   165 170 175
   Ile His His Ile Lys Thr Glu Leu Glu Glu Leu Gly Phe Glu Ala Leu
   180 185 190
   Tyr Pro Asn Arg Tyr Arg Val Ile Lys Glu Val Val Lys Ala Ala Arg
   195 200 205
   Gly Asn Arg Lys Glu Met Ile Gln Lys Ile Leu Ser Glu Ile Glu Gly
   210 215 220
   Arg Leu Gln Glu Ala Gly Ile Pro Cys Arg Val Ser Gly Arg Glu Lys
   225 230 235 240
   His Leu Tyr Ser Ile Tyr Cys Lys Met Val Leu Lys Glu Gln Arg Phe
   245 250 255
   Tyr Ser Ile Met Asp Ile Tyr Ala Phe Arg Val Ile Val Asn Asp Ser
   260 265 270
   Asp Thr Cys Tyr Arg Val Leu Gly Gln Met His Ser Leu Tyr Lys Pro
   275 280 285
   Arg Pro Gly Arg Val Lys Asp Tyr Ile Ala Ile Pro Lys Ala Asn Gly
   290 295 300
   Tyr Gln Ser Leu His Thr Ser Met Ile Gly Pro His Gly Val Pro Val
   305 310 315 320
   Glu Val Gln Ile Arg Thr Glu Asp Met Asp Gln Met Ala Glu Met Gly
   325 330 335
   Val Ala Ala His Trp Ala Tyr Lys Glu His Gly Glu Thr Ser Thr Thr
   340 345 350
   Ala Gln Ile Arg Ala Gln Arg Trp Met Gln Ser Leu Leu Glu Leu Gln
   355 360 365
   Gln Ser Ala Gly Ser Ser Phe Glu Phe Ile Glu Ser Val Lys Ser Asp
   370 375 380
   Leu Phe Pro Asp Glu Ile Tyr Val Phe Thr Pro Glu Gly Arg Ile Val
   385 390 395 400
   Glu Leu Pro Ala Gly Ala Thr Pro Val Asp Phe Ala Tyr Ala Val His
   405 410 415
   Thr Asp Ile Gly His Ala Cys Val Gly Ala Arg Val Asp Arg Gln Pro
   420 425 430
   Tyr Pro Leu Ser Gln Pro Leu Thr Ser Gly Gln Thr Val Glu Ile Ile
   435 440 445
   Thr Ala Pro Gly Ala Arg Pro Asn Ala Ala Trp Leu Asn Phe Val Val
   450 455 460
   Ser Ser Lys Ala Arg Ala Lys Ile Arg Gln Leu Leu Lys Asn Leu Lys
   465 470 475 480
   Arg Asp Asp Ser Val Ser Leu Gly Arg Arg Leu Leu Asn His Ala Leu
   485 490 495
   Gly Gly Ser Arg Lys Leu Asn Glu Ile Pro Gln Glu Asn Ile Gln Arg
   500 505 510
   Glu Leu Asp Arg Met Lys Leu Ala Thr Leu Asp Asp Leu Leu Ala Glu
   515 520 525
   Ile Gly Leu Gly Asn Ala Met Ser Val Val Val Ala Lys Asn Leu Gln
   530 535 540
   His Gly Asp Ala Ser Ile Pro Pro Ala Thr Gln Ser His Gly His Leu
   545 550 555 560
   Pro Ile Lys Gly Ala Asp Gly Val Leu Ile Thr Phe Ala Lys Cys Cys
   565 570 575
   Arg Pro Ile Pro Gly Asp Pro Ile Ile Ala His Val Ser Pro Gly Lys
   580 585 590
   Gly Leu Val Ile His His Glu Ser Cys Arg Asn Ile Arg Gly Tyr Gln
   595 600 605
   Lys Glu Pro Glu Lys Phe Met Ala Val Glu Trp Asp Lys Glu Thr Ala
   610 615 620
   Gln Glu Phe Ile Thr Glu Ile Lys Val Glu Met Phe Asn His Gln Gly
   625 630 635 640
   Ala Leu Ala Asn Leu Thr Ala Ala Ile Asn Thr Thr Thr Ser Asn Ile
   645 650 655
   Gln Ser Leu Asn Thr Glu Glu Lys Asp Gly Arg Val Tyr Ser Ala Phe
   660 665 670
   Ile Arg Leu Thr Ala Arg Asp Arg Val His Leu Ala Asn Ile Met Arg
   675 680 685
   Lys Ile Arg Val Met Pro Asp Val Ile Lys Val Thr Arg Asn Arg Asn
   690 695 700
<210> 4
   <211> 90
   <212> DNA
   <213> Klebsiella pneumoniae
<220>
   <223> CGTase-Signalsequenz
<400> 4
   atgaaaagaa accgtttttt taatacctcg gctgctattg ccatttcgat tgcattaaat 60
   actttttttt gtagcatgca gacgattgct 90
<210> 5
   <211> 90
   <212> DNA
   <213> Artificial Sequence
<220>
   <223> Description of Artificial Sequence: Variante der CGTase-Signalsequenz von K. pneumoniae
<400> 5
   atgaaaagaa accgtttttt taatacctcg gctgctattg ccatttcgat tgcattacag 60
   atcttttttc cgtccgcttc cgctttcgct 90
<210> 6
   <211> 70
   <212> DNA
   <213> Artificial Sequence
<220>

   <223> Description of Artificial Sequence: Oligonukleotid relAmut-fw
<400> 6
   atggttgcgg taagaagtgc acatatcaat aaggctggtg aatttgatcc atgggaatta 60
   gccatggtcc 70
<210> 7
   <211> 70
   <212> DNA
   <213> Artificial Sequence
<220>
   <223> Description of Artificial Sequence: Oligonukleotid relAmut-rev
<400> 7
   ctaactcccg tgcaaccgac gcgcgtcgat aacatccggc acctggttga gtgtaggctg 60
   gagctgcttc 70
<210> 8
   <211> 16
   <212> DNA
   <213> Artificial Sequence
<220>
   <223> Description of Artificial Sequence: Oligonukleotid relA-fw
<400> 8
   cattgtagat acgagc 16
<210> 9
   <211> 15
   <212> DNA
   <213> Artificial Sequence
<220>
   <223> Description of Artificial Sequence: Oligonukleotid relA-rev
<400> 9
   gatttcggca ggtct 15
<210> 10
   <211> 70
   <212> DNA
   <213> Artificial Sequence
<220>
   <223> Description of Artificial Sequence: Oligonukleotid spoTmut-fw
<400> 10
   ttgtatctgt ttgaaagcct gaatcaactg attcaaacct acctgccgga atgggaatta 60
   gccatggtcc 70
<210>
   <211> 70
   <212> DNA
   <213> Artificial Sequence
<220>
   <223> Description of Artificial Sequence:Oligonukleotid spoTmut-rev
<400> 11
<210> 12
   <211> 17
   <212> DNA
   <213> Artificial Sequence
<220>
   <223> Description of Artificial Sequence: Oligonukleotid spoT-fw
<400> 12
   aatcacaaag cgggtcg 17
<210> 13
   <211> 15 <212> DNA
   <213> Artificial Sequence
<220>
   <223> Description of Artificial Sequence: Oligonukleotid spoT-rev
<400> 13
   cctggcgagc atttc 15
<210> 14
   <211> 70
   <212> DNA
   <213> Artificial Sequence
<220>
   <223> Description of Artificial Sequence: Oligonukleotid recG-catfw
<400> 14
   cctaccttcc tgccggatgc gattcatcac cctacaaatt caataaatta atgggaatta 60
   gccatggtcc 70
<210> 15
   <211> 70
   <212> DNA
   <213> Artificial Sequence
<220>
   <223> Description of Artificial Sequence: Oligonukleotid gltS-catrev
<400> 15
   ccgccgcaac aaagacaaat gcctgatacg cttcgcttat caagcctgcg gtgtaggctg 60
   gagctgcttc 70
<210> 16
   <211> 17
   <212> DNA

   <213> Artificial Sequence
<220>
   <223> Description of Artificial Sequence: oligonukleotid recG-fw
<400> 16
   cgtcagacgg gtaatgc 17
<210> 17
   <211> 16
   <212> DNA
   <213> Artificial Sequence
<220>
   <223> Description of Artificial Sequence: Oligonukleotid gltS-rev
<400> 17
   ggcgatggcg ttgatg 16